# EUROPÄISCHE PATENTANMELDUNG

(11) **EP 2 532 413 A1**
(43) Veröffentlichungstag der Anmeldung: **12.12.2012**
(21) Anmeldenummer: 11169501.1
(22) Anmeldetag: 10.06.2011
(51) Int. Cl.: B01D 53/14, B01D 53/62, C07D 211/58, C10L 3/10, F23J 15/04

(54) **Verfahren zur Absorption von CO2 aus einer Gasmischung**

(71) Anmelder: Evonik Degussa GmbH, 45128 Essen (DE)
(72) Erfinder: Rolker, Jörn Dr., 63755 Alzenau (DE); Seiler, Matthias Dr., 64347 Greisheim (DE); Schneider, Rolf, 63584 Gründau-Rothenbergen (DE)

(57) **Zusammenfassung**

Ein Verfahren zur Absorption von CO₂ aus einer Gasmischung durch in Kontakt bringen der Gasmischung bei einem anfänglichen Partialdruck von CO₂ von 0,01 bis 0,6 bar mit einem Absorptionsmedium umfassend Wasser und ein Amin der Formel (I) 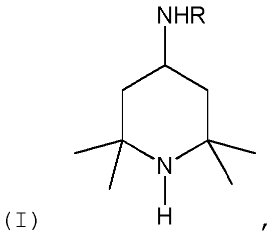 worin R ein n-Alkylrest mit 1 bis 4 Kohlenstoffatomen ist, lässt sich ohne Ausfällung eines Feststoffs bei der Absorption von CO₂ betreiben.

## Beschreibung

Die Erfindung betrifft ein Verfahren zur Absorption von CO₂ aus einer Gasmischung, insbesondere aus einem Verbrennungsabgas.

In zahlreichen industriellen und chemischen Prozessen treten Gasströme auf, die einen unerwünschten Gehalt von CO₂ aufweisen, dessen Gehalt für die weitere Verarbeitung, für den Transport oder für die Vermeidung von CO₂-Emissionen verringert werden muss.

Im industriellen Maßstab werden zur Absorption von CO₂ aus einer Gasmischung üblicherweise wässrige Lösungen von Alkanolaminen als Absorptionsmedium eingesetzt. Das beladene Absorptionsmedium wird durch Erwärmen, Entspannen auf einen niedrigeren Druck oder Strippen regeneriert, wobei das Kohlendioxid desorbiert wird. Nach dem Regenerationsprozess kann das Absorptionsmedium wieder verwendet werden. Diese Verfahren sind zum Beispiel in Rolker, J.; Arlt, W.; "Abtrennung von Kohlendioxid aus Rauchgasen mittels Absorption" in Chemie Ingenieur Technik 2006, 78, Seiten 416 bis 424 sowie in Kohl, A. L.; Nielsen, R. B., "Gas Purification", 5. Aufl., Gulf Publishing, Houston 1997 beschrieben.

Diese Verfahren haben jedoch den Nachteil, dass zur Abtrennung von CO₂ durch Absorption und nachfolgende Desorption relativ viel Energie benötigt wird und dass bei der Desorption nur ein Teil des absorbierten CO₂ wieder desorbiert wird, so dass in einem Zyklus aus Absorption und Desorption die Kapazität des Absorptionsmediums nicht ausreichend ist.

US 7,419,646 beschreibt ein Verfahren zur Entsäuerung von Abgasen, bei dem ein Absorptionsmedium verwendet wird, das bei der Absorption des sauren Gases zwei voneinander trennbare Phasen ausbildet. Als reaktive Verbindung zur Absorption eines sauren Gases wird in Spalte 6 unter anderem 4-Amino-2,2,6,6-tetramethylpiperidin genannt. Das Verfahren von US 7,419,646 hat den Nachteil, dass zusätzliche Apparate für die Trennung der bei der Absorption anfallenden zwei Phasen erforderlich sind.

US 2009/0199709 beschreibt ein ähnliches Verfahren, bei dem nach Absorption des sauren Gases durch Erhitzen des beladenen Absorptionsmediums zwei voneinander trennbare Phasen ausgebildet und voneinander getrennt werden. Auch hier wird als geeignete reaktive Verbindung zur Absorption eines sauren Gases anderem 4-Amino-2,2,6,6-tetramethylpiperidin genannt.

FR 2900841 und US 2007/0286783 beschreiben Verfahren zur Entsäuerung von Abgasen, bei denen aus dem beladenen Absorptionsmedium die mit CO₂ umgesetzte reaktive Verbindung durch Extraktion abgetrennt wird. Als reaktive Verbindung zur Absorption eines sauren Gases wird unter anderem 4-Amino-2,2,6,6-tetramethylpiperidin genannt.

WO 2010/089257 beschreibt ein Verfahren zur Absorption von CO₂ aus einer Gasmischung mit einem Absorptionsmedium, das Wasser und ein 4-Amino-2,2,6,6-tetramethylpiperidin umfasst, wobei das Amin an der 4-Aminogruppe alkyliert sein kann. Bei Absorptionsmedien, die 4-Amino-2,2,6,6-tetramethylpiperidin als Absorptionsmittel enthalten, kommt es allerdings bei der Absorption von CO₂ leicht zur Ausfällung des Carbamatsalzes. WO 2010/089257 beschreibt den Zusatz von Lösungsmitteln, wie Sulfolan oder ionischen Flüssigkeiten, um das Absorptionsmedium einphasig zu halten und eine höhere Absorptionskapazität für CO₂ zu erzielen.

Es besteht deshalb weiterhin ein Bedarf nach einem Verfahren zur Absorption von CO₂ aus einer Gasmischung, das sich zur Absorption von CO₂ aus Verbrennungsabgasen eignet und mit dem sich eine hohe Absorptionskapazität für CO₂ erreichen lässt, wobei sich im Verfahren auch ohne Zusatz eines Lösungsmittels eine Trennung in zwei flüssige Phasen oder die Ausfällung eines Feststoffs bei der Absorption von CO₂ vermeiden lässt.

Es wurde nun gefunden, dass sich diese Aufgabe lösen lässt indem als reaktive Verbindung zur Absorption von CO₂ ein 4-Amino-2,2,6,6-tetramethylpiperidin mit einem Methyl-, Ethyl-, n-Propyl- oder n-Butylsubstituenten an der 4-Aminogruppe eingesetzt wird und der CO₂-Partialdruck der eingesetzten Gasmischung im Bereich von 0,01 bis 0,6 bar gehalten wird.

Gegenstand der Erfindung ist deshalb ein Verfahren zur Absorption von CO₂ aus einer Gasmischung durch in Kontakt bringen der Gasmischung bei einem anfänglichen Partialdruck von CO₂ von 0,01 bis 0,6 bar mit einem Absorptionsmedium umfassend Wasser und ein Amin der Formel (I) 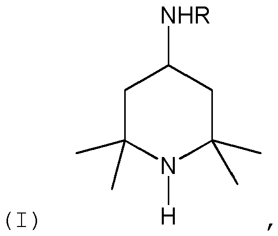 worin R ein n-Alkylrest mit 1 bis 4 Kohlenstoffatomen ist, wobei das Absorptionsmedium kein Amin mit mehr als zwei Stickstoffatomen umfasst.

Das im erfindungsgemäßen Verfahren verwendete Absorptionsmedium umfasst Wasser und ein Amin der Formel (I), wobei R ein n-Alkylrest mit 1 bis 4 Kohlenstoffatomen ist. R kann demnach ein Methylrest, ein Ethylrest, ein n-Propylrest oder ein n-Butylrest sein. Vorzugsweise ist R ein n-Propylrest oder ein n-Butylrest, besonders bevorzugt ein n-Butylrest. Amine der Formel (I) können aus handelsüblichem Triacetonamin durch reduktive Aminierung, d.h. durch Umsetzung von Triacetonamin mit einem Amin der Formel RNH₂ und Wasserstoff in Gegenwart eines Hydrierkatalysators hergestellt werden. Das Absorptionsmedium umfasst vorzugsweise 10 bis 50 Gew.-%, besonders bevorzugt 15 bis 30 Gew.-% Amine der Formel (I).

Das Absorptionsmedium kann zusätzlich zu Wasser und Aminen der Formel (I) noch ein oder mehrere physikalische Lösungsmittel enthalten. Der Anteil an physikalischen Lösungsmitteln kann dabei bis zu 50 Gew.-% betragen. Als physikalische Lösungsmittel eignen sich Sulfolan, aliphatische Säureamide, wie N-Formylmorpholin, N-Acetylmorpholin, N-Alkylpyrrolidone, insbesondere N-Methyl-2-pyrrolidon, oder N-Alkylpiperidone, sowie Diethylenglykol, Triethylenglykol und Polyethylenglykole und deren Alkylether, insbesondere Diethylenglykolmonobutylether. Vorzugsweise enthält das Absorptionsmedium jedoch kein physikalisches Lösungsmittel.

Das Absorptionsmedium kann zusätzlich noch weitere Additive, wie Korrosionsinhibitoren, benetzungsfördernde Additive und Entschäumer aufweisen.

Als Korrosionsinhibitoren können im Absorptionsmedium alle Stoffe verwendet werden, die dem Fachmann zur Absorption von CO₂ unter Verwendung von Alkanolaminen als geeignete Korrosionsinhibitoren bekannt sind, insbesondere die in US 4,714,597 beschriebenen Korrosionsinhibitoren. Die Menge an Korrosionsinhibitoren kann im erfindungsgemäßen Verfahren deutlich geringer als bei einem üblichen, Ethanolamin enthaltenden Absorptionsmedium gewählt werden, da das im erfindungsgemäßen Verfahren verwendete Absorptionsmedium gegenüber metallischen Werkstoffen deutlich weniger korrosiv ist als die üblicherweise verwendeten Ethanolamin enthaltenden Absorptionsmedien.

Als benetzungsförderndes Additiv werden vorzugsweise die aus WO 2010/089257 Seite 11, Zeile 18 bis Seite 13, Zeile 7 bekannten nichtionischen Tenside, zwitterionischen Tenside und kationischen Tenside verwendet.

Als Entschäumer können im Absorptionsmedium alle Stoffe verwendet werden, die dem Fachmann zur Absorption von CO₂ unter Verwendung von Alkanolaminen als geeignete Entschäumer bekannt sind.

Im erfindungsgemäßen Verfahren wird eine CO₂ enthaltende Gasmischung bei einem anfänglichen Partialdruck von CO₂ von 0,01 bis 0,6 bar mit dem Absorptionsmedium in Kontakt gebracht. Vorzugsweise beträgt der anfängliche Partialdruck von CO₂ in der Gasmischung von 0,05 bis 0,6 bar, besonders bevorzugt von 0,1 bis 0,5 bar und am meisten bevorzugt von 0,1 bis 0,2 bar. Der Gesamtdruck der Gasmischung liegt vorzugsweise im Bereich von 0,8 bis 10 bar, besonders bevorzugt 0,9 bis 5 bar.

Die Gasmischung kann ein Erdgas, ein Methan enthaltendes Biogas aus einer Fermentation, Kompostierung oder Kläranlage, ein Verbrennungsabgas, ein Abgas aus einer Kalzinierungsreaktion, wie dem Brennen von Kalk oder der Herstellung von Zement, ein Restgas aus einem Hochofenprozess zur Eisenherstellung oder eine aus einer chemischen Umsetzung resultierende Gasmischung, wie beispielsweise ein Kohlenmonoxid und Wasserstoff enthaltendes Synthesegas oder ein Reaktionsgas einer Wasserstoffherstellung durch Dampfreformieren sein. Vorzugsweise ist die Gasmischung ein Verbrennungsabgas oder eine Gasmischung aus der Vergärung oder Kompostierung von Biomasse, besonders bevorzugt ein Verbrennungsabgas, zum Beispiel aus einem Kraftwerk.

Die Gasmischung kann zusätzlich zu CO₂ noch weitere saure Gase enthalten, wie beispielsweise COS, H₂S, CH₃SH oder SO₂. In einer bevorzugten Ausführungsform enthält die Gasmischung zusätzlich zu CO₂ noch H₂S. Ein Verbrennungsabgas wird vorzugsweise vorher entschwefelt, d.h. SO₂ wird aus der Gasmischung mit einem aus dem Stand der Technik bekannten Entschwefelungsverfahren, vorzugsweise durch eine Gaswäsche mit Kalkmilch, entfernt, bevor das erfindungsgemäße Absorptionsverfahren durchgeführt wird.

Die Gasmischung weist vor dem in Kontakt bringen mit dem Absorptionsmedium vorzugsweise einen Gehalt an CO₂ im Bereich von 0,1 bis 50 Vol-% auf, besonders bevorzugt im Bereich von 1 bis 20 Vol-% und am meisten bevorzugt im Bereich von 10 bis 20 Vol-%.

Die Gasmischung kann zusätzlich zu CO₂ noch Sauerstoff enthalten, vorzugsweise mit einem Anteil von 0,1 bis 25 Vol-% und besonders bevorzugt mit einem Anteil von 0,1 bis 10 Vol-%.

Für das erfindungsgemäße Verfahren können alle zum in Kontakt bringen einer Gasphase mit einer Flüssigphase geeigneten Apparate verwendet werden, um die Gasmischung mit dem Absorptionsmedium in Kontakt zu bringen. Vorzugsweise werden aus dem Stand der Technik bekannte Gaswäscher oder Absorptionskolonnen verwendet, beispielsweise Membrankontaktoren, Radialstromwäscher, Strahlwäscher, Venturi-Wäscher, Rotations-Sprühwäscher Füllkörperkolonnen, Packungskolonnen oder Bodenkolonnen. Besonders bevorzugt werden Absorptionskolonnen im Gegenstrombetrieb verwendet.

Im erfindungsgemäßen Verfahren wird die Absorption von CO₂ vorzugsweise bei einer Temperatur des Absorptionsmediums im Bereich von 10 bis 80°C, besonders bevorzugt 20 bis 50°C, durchgeführt. Bei Verwendung einer Absorptionskolonne im Gegenstrombetrieb beträgt die Temperatur des Absorptionsmediums besonders bevorzugt 30 bis 60°C beim Eintritt in die Kolonne und 35 bis 70°C beim Austritt aus der Kolonne.

In einer bevorzugten Ausführungsform des erfindungsgemäßen Verfahrens wird im Absorptionsmedium absorbiertes CO₂ durch Erhöhen der Temperatur und/oder Verringern des Drucks wieder desorbiert und das Absorptionsmedium nach dieser Desorption von CO₂ wieder zur Absorption von CO₂ verwendet. Vorzugsweise erfolgt die Desorption durch Erhöhen der Temperatur. Durch einen solchen zyklischen Prozess aus Absorption und Desorption kann CO₂ aus der Gasmischung ganz oder teilweise abgetrennt und getrennt von anderen Komponenten der Gasmischung erhalten werden.

Alternativ zum Erhöhen der Temperatur oder dem Verringern des Drucks oder zusätzlich zu einer Temperaturerhöhung und/oder Druckverringerung kann auch eine Desorption durch Strippen des mit CO₂ beladenen Absorptionsmediums mit einem inerten Gas, wie zum Beispiel Luft oder Stickstoff, durchgeführt werden.

Wenn bei der Desorption von CO₂ zusätzlich auch Wasser aus dem Absorptionsmedium entfernt wird, kann dem Absorptionsmedium vor der Wiederverwendung zur Absorption gegebenenfalls noch Wasser zugesetzt werden.

Für die Desorption können alle Apparate verwendet werden, die aus dem Stand der Technik zur Desorption eines Gases aus einer Flüssigkeit bekannt sind. Vorzugsweise wird die Desorption in einer Desorptionskolonne durchgeführt. Alternativ kann die Desorption von CO₂ auch in einer oder mehreren Flash-Verdampfungsstufen durchgeführt werden.

Die Desorption wird vorzugsweise bei einer Temperatur im Bereich von 30 bis 180°C durchgeführt. Bei einer Desorption durch Erhöhen der Temperatur wird die Desorption von CO₂ vorzugsweise bei einer Temperatur des Absorptionsmediums im Bereich von 50 bis 180°C, besonders bevorzugt 80 bis 150°C, durchgeführt. Die Temperatur bei der Desorption liegt dabei vorzugsweise mindestens 20 °C, besonders bevorzugt mindestens 50 °C, oberhalb der Temperatur bei der Absorption.

Da das im erfindungsgemäßen Verfahren verwendete Absorptionsmedium eine hohe Absorptionskapazität für CO₂ bei einer für eine technische Anwendung ausreichend hohen Absorptionsrate aufweist und im erfindungsgemäßen Verfahren als homogene Lösung vorliegt, ohne dass es bei Absorption von CO₂ zur Ausfällung eines Feststoffs kommt, kann das erfindungsgemäße Verfahren in einfach aufgebauten Anlagen eingesetzt werden und erreicht dabei eine im Vergleich zu Ethanolamin verbesserte Absorptionsleistung für CO₂. Gleichzeitig wird im Vergleich zu Ethanolamin wesentlich weniger Energie zur Desorption von CO₂ benötigt.

In einer bevorzugten Ausführungsform des erfindungsgemäßen Verfahrens erfolgt die Desorption durch Strippen mit einem Inertgas, wie beispielsweise Luft oder Stickstoff, in einer Desorptionskolonne. Das Strippen in der Desorptionskolonne erfolgt vorzugsweise bei einer Temperatur des Absorptionsmediums im Bereich von 60 bis 100°C. Durch das Strippen kann mit geringem Energiebedarf ein niedriger Restgehalt des Absorptionsmediums an CO₂ nach Desorption erreicht werden.

In einer weiteren Ausführungsform wird die Zusammensetzung des Absorptionsmediums so gewählt, dass bei einer Temperaturanhebung zur Desorption eine Entmischung des mit CO₂ beladenen Absorptionsmittels in eine wässrige CO₂-reiche und eine organische CO₂-arme flüssige Phase erfolgt. Dies ermöglicht eine Regenerierung bei niedrigeren Temperaturen und eine Einsparung von Energie bei der Regenerierung, indem nur die CO₂-reiche Phase regeneriert wird und die CO₂-arme direkt in die Absorption zurückgeführt wird. In diesen Fällen kann bereits ein energetisch günstiger Flash ausreichend sein, um das mit CO₂ beladene Absorptionsmittel zu regenerieren.

Die nachfolgenden Beispiele verdeutlichen die Erfindung, ohne jedoch den Gegenstand der Erfindung zu beschränken.

### Beispiele

Die untersuchten Absorptionsmedien sind in Tabelle 1 zusammengestellt.

Zur Bestimmung der CO₂-Beladung, des CO₂-Hubs und der relativen Absorptionsrate wurden 150 g Absorptionsmedium in einem thermostatisierbaren Behälter mit aufgesetztem und auf 3°C gekühltem Rückflusskühler vorgelegt. Nach Aufheizen auf 40°C bzw. 100°C wurde über eine Fritte am Behälterboden eine Gasmischung aus 14 Vol-% CO₂, 80 Vol-% Stickstoff und 6 Vol-% Sauerstoff mit einer Flussrate von 59 1/h durch das Absorptionsmedium geleitet und die CO₂-Konzentration im aus dem Rückflusskühler austretenden Gasstrom über die IR-Absorption mit einem CO₂-Analysator bestimmt. Durch Integration der Differenz des CO₂-Gehalts im eingeleiteten Gasstrom und im austretenden Gasstrom wurde die aufgenommene CO₂-Menge ermittelt und die Gleichgewichtsbeladung des Absorptionsmediums mit CO₂ berechnet. Der CO₂-Hub wurde als Differenz der bei 40°C und 100°C aufgenommenen CO₂-Mengen berechnet. Aus der Steigung der Kurve der CO₂-Konzentration im austretenden Gasstrom für den Konzentrationsanstieg von 1 Vol-% auf 12 Vol-% wurde eine relative Absorptionsrate von CO₂ im Absorptionsmedium bestimmt. Die so bestimmten Gleichgewichtsbeladungen bei 40 und 100°C in mol CO₂ / mol Amin, der CO₂-Hub in mol CO₂ / kg Absorptionsmedium und die auf Beispiel 1 mit 100 % bezogene relative Absorptionsrate von CO₂ sind in Tabelle 1 angeführt.

In Beispiel 2 kam es bei der Beladung mit CO₂ bei 40°C zur Ausfällung des Carbamatsalzes von TAD (4-Amino-2,2,6,6-tetramethylpiperidin).

**Tabelle 1**

| Beispiel | | 1* | 2* | 3 | 4 | 5 |
|---|---|---|---|---|---|---|
| Anteile in Gew.-% | | | | | | |
| | Wasser | 70 | 70 | 70 | 70 | 70 |
| | MEA | 30 | 0 | 0 | 0 | 0 |
| | TAD | 0 | 30 | 0 | 0 | 0 |
| | Me-TAD | 0 | 0 | 30 | 0 | 0 |
| | Pr-TAD | 0 | 0 | 0 | 30 | 0 |
| | Bu-TAD | 0 | 0 | 0 | 0 | 30 |
| Beladung bei 40°C in mol/mol | | 0,45 | 1,08 | ** | 1,53 | 1,38 |
| Beladung bei 100°C in mol/mol | | 0,22 | 0,54 | ** | 0,39 | 0,20 |
| CO₂-Hub in mol/kg | | 1,15 | 1, 04 | ** | 1,71 | 1,66 |
| Relative Absorptionsrate in % | | 100 | 178 | ** | 41 | 50 |

| | | | | | | |
|---|---|---|---|---|---|---|
| * nicht erfindungsgemäß ** beim Begasen fiel Feststoff aus MEA: Ethanolamin TAD: 4-Amino-2,2,6,6-tetramethylpiperidin Me-TAD: 4-Methylamino-2,2,6,6-tetramethylpiperidin Pr-TAD: 4-(n-Propylamino)-2,2,6,6-tetramethylpiperidin Bu-TAD: 4-(n-Butylamino)-2,2,6,6-tetramethylpiperidin | | | | | | |

Die bei einem CO₂-Partialdruck von 0,14 bar durchgeführten Beispiele zeigen, dass sich mit dem erfindungsgemäßen Verfahren ein höherer CO₂-Hub erzielen lässt, als bei Verfahren, die Ethanolamin oder TAD zur Absorption einsetzen.

Zur Bestimmung des CO₂-Partialdrucks, ab dem es bei der Absorption zur Ausfällung eines Feststoffs kommt, wurde Absorptionsmedium mit der in Tabelle 2 angeführten Zusammensetzung in einem thermostatisierbaren Behälter mit aufgesetztem und auf 3°C gekühltem Rückflusskühler vorgelegt. Nach Aufheizen auf 40°C wurde über eine Fritte bei 1 bar für 2 h eine Gasmischung aus CO₂ und Stickstoff durch das Absorptionsmedium geleitet, wobei Gasmischungen mit 20, 40, 60 und 80 Vol-% CO₂ eingesetzt wurden um CO₂-Partialdrücke in der zugeführten Gasmischung von 0,2 bis 0,8 bar einzustellen. In Tabelle 2 ist zusammengefasst, in welchen der Versuche ein Ausfallen von Feststoff beobachtet wurde.

Mit dem Arbeitsmedium von Beispiel 2 wurde außerdem ein Versuch durchgeführt, bei dem in einem thermostatisierten 500 ml Autoklav 250 g Arbeitsmedium bei 40 °C vorgelegt wurde und nach evakuieren CO₂ druckgeregelt bis zur Sättigung zudosiert wurde, wobei der stufenweise erhöht wurde um CO₂-Partialdrücke von 75 mbar, 90 mbar und 100 mbar einzustellen. Bei einem CO₂-Partialdruck von 100 mbar wurde die Ausfällung von Feststoff beobachtet.

**Tabelle 2**

| Beispiel | | 2* | 3 | 6 | 7 | 4 | 8 | 9 | 5 | 10 | 11 |
|---|---|---|---|---|---|---|---|---|---|---|---|
| Anteile in Gew.-% | | | | | | | | | | | |
| | Wasser | 70 | 70 | 60 | 50 | 70 | 60 | 50 | 70 | 60 | 50 |
| | TAD | 30 | 0 | 0 | 0 | 0 | 0 | 0 | 0 | 0 | 0 |
| | Me-TAD | 0 | 30 | 40 | 50 | 0 | 0 | 0 | 0 | 0 | 0 |
| | Pr-TAD | 0 | 0 | 0 | 0 | 30 | 40 | 50 | 0 | 0 | 0 |
| | Bu-TAD | 0 | 0 | 0 | 0 | 0 | 0 | 0 | 30 | 40 | 50 |

| Feststoffausfällung bei CO₂-Anteil | | | | | | | | | | | |
|---|---|---|---|---|---|---|---|---|---|---|---|
| | 20 Vol-% | ja | nein | nein | ja | nein | ja | ja | nein | nein | nein |
| | 40 Vol-% | ja | ja | ja | ja | ja | ja | ja | nein | nein | nein |
| | 60 Vol-% | ja | ja | ja | ja | ja | ja | ja | nein | nein | nein |
| | 80 Vol-% | ja | ja | ja | ja | ja | ja | ja | ja | ja | ja |

| | | | | | | | | | | | |
|---|---|---|---|---|---|---|---|---|---|---|---|
| *nicht erfindungsgemäß | | | | | | | | | | | |

## Patentansprüche

1. Verfahren zur Absorption von CO₂ aus einer Gasmischung durch in Kontakt bringen der Gasmischung bei einem anfänglichen Partialdruck von CO₂ von 0,01 bis 0,6 bar mit einem Absorptionsmedium umfassend Wasser und ein Amin der Formel (I) 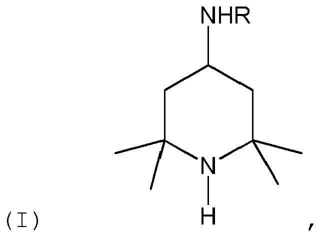 worin R ein n-Alkylrest mit 1 bis 4 Kohlenstoffatomen ist, wobei das Absorptionsmedium kein Amin mit mehr als zwei Stickstoffatomen umfasst.

2. Verfahren nach Anspruch 1,
**dadurch gekennzeichnet,**
**dass** R ein n-Butylrest ist.

3. Verfahren nach Anspruch 1 oder 2,
**dadurch gekennzeichnet,**
**dass** der anfängliche Partialdruck von CO₂ in der Gasmischung von 0,1 bis 0,5 bar beträgt.

4. Verfahren nach einem der Ansprüche 1 bis 3,
**dadurch gekennzeichnet,**
**dass** der anfängliche Anteil an CO₂ in der Gasmischung von 0,1 bis 50 Vol-% beträgt.

5. Verfahren nach einem der Ansprüche 1 bis 4,
**dadurch gekennzeichnet,**
**dass** die Gasmischung von 0,1 bis 25 Vol-% Sauerstoff enthält.

6. Verfahren nach einem der Ansprüche 1 bis 5,
**dadurch gekennzeichnet,**
**dass** die Gasmischung ein Verbrennungsabgas ist.

7. Verfahren nach einem der Ansprüche 1 bis 5,
**dadurch gekennzeichnet,**
**dass** die Gasmischung aus der Vergärung oder Kompostierung von Biomasse stammt.

8. Verfahren nach einem der Ansprüche 1 bis 7,
**dadurch gekennzeichnet,**
**dass** das Absorptionsmedium 10 bis 50 Gew.-% eines Amins der Formel (I) umfasst.

9. Verfahren nach einem der Ansprüche 1 bis 8,
**dadurch gekennzeichnet,**
**dass** das Absorptionsmedium kein Lösungsmittel enthält.

10. Verfahren nach einem der Ansprüche 1 bis 9,
**dadurch gekennzeichnet,**
**dass** im Absorptionsmedium absorbiertes CO₂ durch Erhöhen der Temperatur und/oder Verringerung des Drucks wieder desorbiert wird und das Absorptionsmedium nach dieser Desorption von CO₂ wieder zur Absorption von CO₂ verwendet wird.

11. Verfahren nach Anspruch 10,
**dadurch gekennzeichnet,**
**dass** die Absorption bei einer Temperatur im Bereich von 10 bis 80°C und die Desorption bei einer Temperatur im Bereich von 30 bis 180°C durchgeführt wird.

12. Verfahren nach Anspruch 10 oder 11,
**dadurch gekennzeichnet,**
**dass** mit CO₂ beladenes Absorptionsmedium zur Desorption mit einem inerten Gas gestrippt wird.
